# EUROPEAN PATENT APPLICATION

(11) **EP 2 319 536 A1**
(43) Date of publication of application: **11.05.2011**
(21) Application number: 11153818.7
(22) Date of filing: 11.02.2005
(51) Int. Cl.: A61K 39/395, A61K 49/00, C07K 16/00, G01N 33/53, G01N 33/574, A61P 35/00, A61P 37/00, C07K 16/28

(54) **Inhibiting CAV3 isoforms and the delta25B splice varients for the diagnosis and treatment of cancer**

(30) Priority: 11.02.2004 US 543756 P
(62) Divisional of application: 05713305.0
(71) Applicant: UNIVERSITY OF VIRGINIA PATENT FOUNDATION, Charlottesville, VA 22902 (US)
(72) Inventor: Gray, Lloyd S., Charlottesville, VA 22911 (US); MacDonald, Timothy L., Charlottesville, VA 22902 (US)
(74) Representative: HOFFMANN EITLE

(57) **Abstract**

The present application is directed to an antibody that specifically binds to a Cav3 isoform or its δ25 splicing variants thereof.

## Description

### BACKGROUND OF THE INVENTION

Ca2+ entry is critical for cellular proliferation. Because unusually rapid proliferation is a hallmark of cancer, elucidation of the mechanism of Ca2+ entry is of scientific importance with potential clinical significance. However, the mechanism of Ca2+ entry in electrically non-excitable cells remains elusive. The majority of cancers arise from cell types considered "electrically non-excitable." This is to distinguish the means of regulation of Ca2+ entry in these cells from those having action potentials, which are "electrically excitable" cells. In electrically excitable cells, an action potential opens voltage gated Ca2+ channels that admit the Ca2+ required for initiating events such as neurosecretion. In electrically non-excitable cells, the regulatory mechanism mediating Ca2+ entry is unclear in part because of the uncertainty about the molecular mechanism by which Ca+ entry occurs.

The two types of ion channels or transporters most commonly implicated in capacitative Ca2+ entry are I_{CRAC} and various members of the Trp, or vanilloid, family of Ca2+ channel. These channels are generally non-selective cation channels that lack voltage dependent gating. I_{CRAC} is selective for Ca2+ over other cations but its molecular identity has remained elusive and its participation in proliferation questioned. Consequently, it has been difficult to tie these various proteins' function to capacitative Ca2+ entry. This may reflect a particular complexity of Ca2+ signaling in electrically non-excitable cells arising from participation of numerous channels and transporters in this process. It is also possible that the primary means of Ca2+ entry in electrically non-excitable cells has yet to be fully elucidated.

Knowledge about voltage gated calcium channels in electrically excitable cells has been exploited profitably. Pharmacological modulation of these channels' function is tremendously important in the practice of medicine; for example, calcium channel blockers are in widespread use in the treatment of epilepsy, hypertension, and angina pectoris. Unfortunately, such intervention is not yet available for calcium channels in electrically non-excitable cells. This deficiency likely reflects the fact that the mechanism by which calcium entry occurs has not been clearly identified.

The present invention takes the following approach to dissecting the Ca2+ entry pathway in electrically non-excitable cells. Taking advantage of Ca2+ entry blockade by Ni2+, as measured by fluorescence techniques (Merritt and Rink J.Biol.Chem. 262:17362-69, 1987) compounds in the published literature with a similar ability was identified. The structure/activity relationship of these know compounds was used to guide the synthesis of novel compounds with enhanced potency to block Ca2+ entry into the cells and proliferation of several cancer cell lines. Administration of one of these compounds, TH-1177, significantly extended the lifespan of SCID mice inoculated with human PC3 prostate cancer cells. The present invention demonstrates that two representative compounds block the Ca2+ current through the heterologously expressed Cav3.2 isoform of T type Ca2+ channels and inhibit proliferation of HEK293 cells stably transfected with this isoform, These two compounds block the Ca2+ current and capacitative Ca2+ entry with similar potencies and identical stereoselectivity. Importantly, cell lines sensitive to the novel compounds express message for Cav3.2, its δ25B splice variant, or both, while a cell line resistant to the compounds does not detectably express either message.

The library of compounds that the present invention has developed has broad activity against cancer cell lines from various tissues. They act cytostatically and are equally potent at inhibiting hormone sensitive and insensitive breast and prostate cancer lines. It has been demonstrated that the target of these-compounds' anti-proliferative activity is the Ca+ channel isoform Cav3.2 and/or its δ25 splice variant. A method of determining the expression of Cav3.2 and δ25 would therefore have great importance in the diagnosis of cancer.

Mibefradil is a calcium antagonist that is selective for the T-type voltage-operated calcium channel rather than the L type.

Traditionally, chemical agents that interact with molecular targets have been used in chemotherapy. More recently, however, antibodies directed against a molecular target have been developed and used to treat cancer. This same approach can be used with antibodies that inhibit calcium entry in cancer cells by interacting with Cav3.2 or its δ25 splice variant. This inhibition either would occur by direct interference with calcium entry or by antibody mediated down-regulation of the calcium channel protein.

Antibodies, both monoclonal and polyclonal, have demonstrated utility in the diagnosis of numerous diseases including cancer. Because of the involvement of Cav 3.2 and 825 in cancer, monoclonal and polyclonal, antibodies against these proteins would have diagnostic utility.

Antibodies have also shown therapeutic utility in treating cancer. For - example, Herceptin® is used to treat Her2 expressing cancers, primarily of the breast. Antibodies directed at Cav3.2 and/or δ25 could function therapeutically in two ways. The most obvious mechanism of action would be an antibody, whether polyclonal, monoclonal, humanized monoclonal, or an effective fragment of any of the foregoing, that blocked the ability of Cav3.2 or δ25 to admit Ca2+ into the interior of cancer cells thereby inhibiting the proliferation of them. Alternatively, such antibodies or fragments thereof could induce Ca2+ entry thereby causing the apoptotic death of cancer cells.

### SUMMARY OF THE INVENTION

During normal growth and development, cellular proliferation is ubiquitous, while in healthy adults cellular proliferation is limited to only a few tissues such as the bone marrow. Calcium, is required for progression through the cell cycle and cellular proliferation. The present invention identifies that this required calcium entry is mediated by a calcium channels called Cav3 isoform, preferably, Cav3.l., Cav3.2 or Cav3.3 isoform and/or its δ25 splice variant thereof more preferably, Cav3.2 isoform and/or its δ25 splice variant thereof. Pathological cellular proliferation, or cancer, in adults is associated with the functional or physical re-expression of Cav3 isoforms, particularly, Cav3.2 or the δ25 splice variant. Consequently, inhibition of calcium entry by administering chemicals that block Cav3 isoforms, preferably, Cav3.1, Cav3.2 or Cav3.3 isoform and/or its δ25 splice variant thereof, more preferably, Cav3.2 isoform and/or its δ25 splice variant thereof, prevents or inhibits proliferation or growth of cancer cells.

Accordingly, one aspect of the present invention is directed to a method of inhibiting cancer cell growth or proliferation comprising administering to a patient in need thereof a therapeutically effective amount af T type calcium channel selective inhibitor.

Another aspect of the present invention is directed to method for preventing proliferation of cancer cells comprising administering to a patient in need thereof a therapeutically effective amount of T type calcium channel selective inhibitor.

A further aspect of the present invention is directed to a method for treating cancer comprising administering to a patient in need thereof a therapeutically effective amount of T type calcium channel selective inhibitor and a pharmaceutical carrier.

A still further aspect of the present invention is directed to a pharmaceutical composition comprising an antibody that specifically binds or reacts to Cav3 isoforms or their δ25 variants, particularly, Cav3.2 or its δ25 splice variants, and methods of using such compositions for diagnosing and treating cancer.

An aspect of the present invention is to provide methods for inhibiting calcium entry into electrically non-excitable cells with a T type calcium channel selective inhibitor.

Another aspect of the present invention is to provide methods for preventing proliferation of electrically non-excitable cells, with a T type calcium channel selective inhibitor.

Still another aspect of the present invention is to provide methods of treating autoimmune diseases comprising administering to a patient in need thereof with a therapeutically effective amount of T type calcium channel selective inhibitor.

One aspect of the present invention is directed to a method for preventing graft rejections comprising administering to a patient in need thereof with a therapeutically effective amount of T type calcium channel selective inhibitor.

Another aspect of the present invention is directed to a method for preventing apoptosis comprising administering to a patient in need thereof with a therapeutically effective amount of T type calcium channel selective inhibitor.

### BRIEF DESCRIPTION OF THE DRAWING

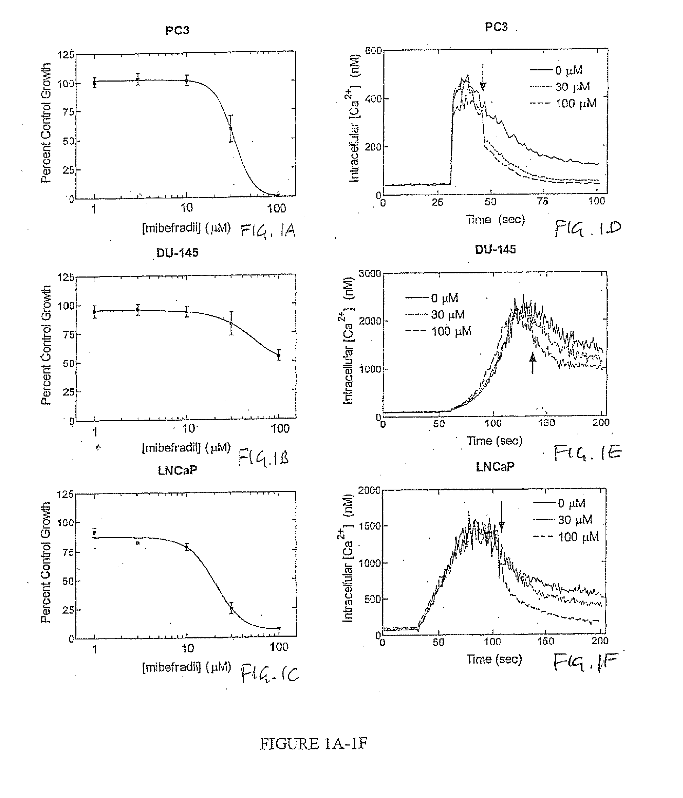

Figures 1A-1F depict that mibefradil inhibited stimulated calcium entry of cancer. cells concordantly with inhibition of proliferation of the cancer cells.

### DETAILED DESCRIPTION OF THE INVENTION

In describing and claiming the invention, the following terminologies are used in accordance with the definitions set forth below.

As used herein, the term "nucleic acid" encompasses RNA as well as single and double-stranded DNA and cDNA. Furthermore, the terms, "nucleic acid," "DNA," "RNA" and similar terms also include nucleic acid analogs, i.e. analogs having other than a phosphodiester backbone. For example, the so-called "peptide nucleic acids," which are known in the art and have peptide bonds instead of phosphodiester bonds in the backbone, are considered within the scope of the present invention.

The term "peptide" encompasses a sequence of 3 or more amino acids wherein the amino acids are naturally occurring or synthetic (non-naturally occurring) amino acids. Peptide mimetics include peptides having one or more of the following modifications:
1. peptides wherein one or more of the peptidyl -- C(O)NR-- linkages (bonds) have been replaced by a non-peptidyl linkage such as a —CH2-carbamate lineage (--CH2OC(O)NR--), a phosphonate linkage, a -CH2-sulfonamide (-CH 2--S(O)2NR-- ) linkage, a urea (-NHC(O)NH-) linkage, a --CH2 -secondary amine linkage, or with an alkylated peptidyl linkage (--C(O)NR--) wherein R is C1-C4 alkyl;
2. peptides wherein the N-terminus is derivatized to a --NRR1 group, to a -- NRC(O)R group, to a --NRC(O)OR group, to a --NRS(O)2R group, to a-NHC(O)NHR group where R and R1 are hydrogen or C1-C4 alkyl with the proviso that R and R1 are not both hydrogen;
3. peptides wherein the C terminus is derivatized to --C(O)R2 where R 2 is selected from the group consisting of C1-C4 alkoxy, and --NR3R4 where R3 and R4 are independently selected from the group consisting of hydrogen and C1-C4 alkyl

Naturally occuring amino acid residues in peptides are abbreviated as recommended by the IUPAC-IUB Biochemical Nomenclature Commission as follows: Phenylalanine is Phe or F; Leucine is Leu or L; Isoleucine is Ile or I; Methionine is Met or M; Norleucine is Nle; Valine is Val or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic Acid is Asp or D; Glutamic Acid.is Glu or E; Cysteine is Cys or C; Tryptophan is Trp or W; Arginine is Arg or R; Glycine is Gly or G, and X is any amino acid. Other naturally occurring amino acids include, by way of example, 4-hydroxyproline, 5-hydroxylysine, and the like.

As used herein, the term "conservative amino acid substitution" is defined herein as exchanges within one of the following five groups:
I. Small aliphatic, nonpolar or slightly polar residues:
   Ala, Ser, Thr, Pro, Gly;
II. Polar, negatively charged residues and their amides:
   Asp, Asn, Glu, Gln;
III, Polar, positively charged residues:
   His, Arg, Lys;
IV. Large, aliphatic, nonpolar residues:
   Met Leu, Ile, Val, Cys
V. Large, aromatic residues:
   Phe, Tyr, Trp

As used herein, the term ''purified'' and like terms relate to the isolation of a molecule or compound in a form that is substantially free of contaminants normally associated with the molecule or compound in a native or natural environment.

As used herein, the term "treating" includes alleviating the symptoms associated with a specific disorder or condition and/or preventing or eliminating said symptoms. For example, treating cancer includes preventing or slowing the growth and/or division of cancer cells as well as killing cancer cells. The term "treating" in the present invention also refers to ameliorating a disease such that the condition of the patient improves or such that the progess of the disease is slowed. The beneficial effects of the T type calcium channel selective inhibitor of the present invention can be determined by monitoring an improvement in one or more symptoms; for example, by monitoring the decrease in proliferation (cancer), by monitoring the increased stablization of a graft in a patient (graft rejection), by monitoring the decreased joint swelling, inflammation, and pain in a patient (rheumatoid arthritis), by monitoring the decrease in insulin requirement (juvenile onset diabetes mellitus), by monitoring the normalization of circulating levels of thyroid hormones in a patient (thyroiditis), by monitoring the decrease in nephritis (systemic lupus erythematosus), etc. Alternatively, the effects of the T type calcium channel selective inhibitor of the present invention can be monitored by comparing them against compounds conventionally used in the treatment of these diseases.

The term "therapeutically effective amount" refers to an amount of the T type calcium channel selective inhibitor compound that is necessary to achieve a desired endpoint (e.g., inhibiting cancer cell proliferation; treating, preventing or controlling cancer in a patient.).

As used herein, the term "pharmaceutically acceptable carrier" includes any of the standard pharmaceutical carriers, such as a phosphate buffered saline solution, water, emulsions such as an oil/water or water/oil emulsion, and various types of wetting agents. The term, also encompasses any of the agents approved by a regulatory agency of the US Federal government or listed in the US Pharmacopeia for use in animals, including humans.

As used herein, the term "antibody" refers to a polyclonal or monoclonal antibody or a binding fragment thereof such as Fab, F(ab')2 and Fv fragments.

One embodiment of the present invention is directed to a method of inhibiting cancer cells comprising administering to a patient in need thereof a therapeutically effective amount of T type calcium channel selective inhibitor. According to the present invention, the T type calcium channel includes, but is not limited to, Cav3 isoform or their δ25 splice variants thereof, preferably, a Cav3.1, Cav3.2 or Cav3.3 isoform and/or its δ25 splice variant thereof, more preferably, Cav3.2 isoform and/or its δ25 splice variant thereof. The dosage of T type calcium channel selective inhibitor contemplated by the present invention can be about 10 mg to about 1000 mg. A preferred dosage of the T type calcium channel selective inhibitor is in an amount resulting in a plasma concentration of about 1 µM to about 100 µM.

Inhibition or blockade of calcium entry slows or stop the division or proliferation of any cancer ordinarily called a solid tumor. Consequently, the pharmaceutical compositions described herein can be used to prevent, control, or treat any cancer of the type ordinarily referred to as a solid tumor or conditions related to such disorders, These solid tumors include, but are not limited to, cancers of the head and ILeGIC, esophagus, stomach, liver, lung, connective tissue, skin, pancreas, intestine, Kidney, breast, ovary, testes, and prostate.

Another embodiment of the present invention is directed to method for preventing proliferation of cancer cells comprising administering a patient in need thereof a therapeutically effective amount of T type calcium channel selective inhibitor.

A further embodiment of the present invention is directed to a methods for treating cancer comprising administering to a patient in need thereof a T type calcium channel selective inhibitor and a pharmaceutical carrier.

According to the present invention, the T type calcium channel selective inhibitor is present in an amount effective to inhibit, prevent or control the proliferation of electrically non-excitable cells, particularly, cancer or tumor cells.

In a preferred embodiment of the present invention, the T type calcium channel selective inhibitor is a tetrahydronaphthalene derivative of the formula 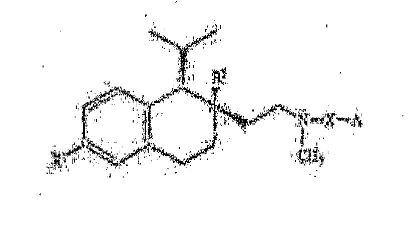 wherein R¹ is a halogen, R² is a lower-alkoxy-lower-alkyl-carbonyloxy, X is a C₂-C₈-alkylcne, and A is a benzimidazolyl optionally substituted at the N atom with 1 to 12 C atoms in the form of their free bases, their hydrates, or their pharmaceutically usable salts for the treatment, control, and prevention of cancer.

A particular preferred T type calcium channel selective inhibitor is mibefradil that has the formula of (1S,2S)-(2 {[3 -(2-benzimidazolyl) propyl] methylamino ethyl)-6-fluoro-1,2,3,4-t-etrahydro-1-isopropyl-2-naphthylmethoxyacetate dihydrochloride.

Mibefradil is a well-known T type voltage gated calcium channel blocker (Massie, B.M. Am. J. Cardia. 80:231, 1997). It is also been shown that blockade of voltage gated calcium in cancerous, electrically non-excitable cells, which constitute solid tumors, inhibits proliferation (U.S. Patent No. 6,413,967, incorporated herein by reference). Consequently, mibefradil inhibits proliferation of electrically non-excitable cancer cells by inhibiting calcium entry into them, as shown in Figures 1A to 1F.

In another embodiment, the T type calcium channel selective inhibitor is an antibody. According to the present invention, since expression of Cav3 isoforms or their δ25 splicing variants is necessary for calcium entry in many types of cancer, antibodies against these proteins will be important in cancer diagnosis. These diagnostic antibodies will be used to establish that a particular cancer in a specific patient has the phenotype that confers sensitivity either to therapeutic antibodies or to chemotherapeutic agents directed toward Cav3 isoforms or their δ25 variants, preferably, a Cav3.1, Cav3.2 or Cav3.3 isoform and/or its δ25 splice variant thereof, more preferably, Cav3.2 isoform and/or its δ25 splice variant thereof.

Accordingly, one embodiment of the present invention is directed to a pharmaceutical composition comprising an antibody that specifically binds or reacts to Gav3 isoforms or their δ25 variants, preferably, a Cav3.1, Cav3.2 or Cav3.3 isoform and/or its δ25 splice variant thereof, more preferably, Cav3.2 isoform and/or its δ25 splice variant thereof; and directed to methods of using such compositions for diagnosing and treating cancer.

In accordance with one embodiment, an improved diagnostic test for neoplastic disease is provided, comprising the steps of contacting a tissue sample with an antibody that specifically binds to Cav3.2 or its δ25 splice variant and determining the relative amount of specifically bound antibody. In one embodiment an antibody is selected that binds to the Cav3.2 protein (UniGene cluster Hs.122359) and/or its δ25B splice variant (GenBank accession number AF223563).

In one embodiment, the antibody to a Cav3 isoform protein and/or its δ25B splice variant, preferably, a Cav3.1, Cav3.2 or Cay3.3 isoform and/or its δ25 splice variant thereof more preferably, Cav3.2 isoform and/or its δ25 splice variant thereof, is a monoclonal antibody. Monoclonal antibody production may be effected using techniques well-known to those skilled in the art. In accordance with the present invention, the process involves first obtaining immune cells (lymphocytes) from the spleen of a mammal (e.g., mouse) which has been previously immunized with the antigen of interest either *in vivo* or *in vitro.* The antibody-secreting lymphocytes are then fused with myeloma cells or transformed cells, which are capable of replicating indefinitely in cell culture, thereby producing an immortal, immunoglobulin-secreting cell line. The resulting fused cells, or hybridomas, are cultured, and the resulting colonies screened for the production of the desired Monoclonal antibodies. Colonies producing such antibodies are cloned, and grown either *in vivo or in vitro* to produce large quantities of antibody. One embodiment of the invention is directed to a hybridoma cell line which produces monoclonal antibodies which bind one of the target antigens of the Cav3.2 protein or its δ5B splice variant. A description of the theoretical basis and practical methodology of fusing such cells is set forth in Kohler and Milstein, Nature, 256:495 (1975), which is hereby incorporated by reference.

In addition to whole antibodies, fragments of antibodies can retain binding specificity for a particular antigen Antibody fragments can be generated by several methods, including, but not limited to proteolysis or synthesis using recombinant DNA technology. An example of such an embodiment is selective proteolysis of an antibody by papain to generate Fab fragments, or by pepsin to generate a F(ab')2 fragment. These antibody fragments can be made by conventional procedures, as described in J. Goding, Monoclonal Antibodies: Principles and Practice, pp. 98-118 [N.Y. Acad,emic Press 1983), which is hereby incorporated by reference. Other fragments of the present antibodies which retain the specific binding of the whole antibody can be generated by other means known to those skilled in the art.

The antibodies or antibody fragments of the present invention can be combined with a carrier or diluent to form a composition. These compositions can be used in standard Molecular Biology techniques such as Western blot analyses, immunofluorescence, and immunoprecipitation. The antibodies of the present invention can also be linked to a detectable label using standard reagents and techniques known to those skilled in the art. For example, see Wensel and Meares, Radioimmunoimaging and Radioimmunotherapy, Elsevier, New York (1983), which is hereby incorporated by reference, for techniques relating to the radiolabeling of antibodies. See also, D. Colcher et al., "Use of Monoclonal Antibodies as Radiopharmaceuticals for the Localization of Human Carcinoma Xeno grafts in Athymic Mice," Meth. EnzvmQL, 121: 802-816 (1986), which is hereby incorporated by reference,

In accordance with one embodiment the antibodies of the present invention are labeled for use in diagnostics or therapeutics. It is not intended that the present invention be limited to any particular detection system or label. The antibody may be labeled with a radioisotope, such as 35S, 1311,I1In, 123I, 99mTc, 32P, 125I, 3H, 14C, and 188Rh, or a non-isotopic labeling reagent including fluorescent labels, such as fluorescein and rhodamine, or other non-isotopic labeling reagents such as biotin or digoxigenin. Antibodies containing biotin may be detected using "detection reagents" such as avidin conjugated to any desirable label such as a fluorochrome. Additional labels suitable for use in accordance with the present invention include nuclear magnetic resonance active labels, electron dense or radiopaque materials, positron emitting isotopes detectable by a positron emission tomography ("PET") scanner, chemilluminescers such as luciferin, and enzymatic markers such as peroxidase or phosphatase.

In one embodiment the antibodies of the present invention are detected through the use of a secondary antibody, wherein the secondary antibody is labeled and is specific for the primary antibody. Alternatively, the antibodies of the present invention can be directly labeled with a radioisotope or fluorochrome such as FITC or rhodamine; in such cases secondary detection reagents may not be required for the detection of the labeled probe. In accordance with one preferred embodiment the antibody is labeled with a fluorophore or chromophore using standard moieties known in the art.

In accordance with one embodiment of the present invention a diagnostic method for detecting cancer is provided. The method comprises the step of detecting the presence of a Cav3 protein and/or its δ25B splice variant, preferably, a Cav3.1, Cav3.2 or Cav3.3 isoform protein and/or its δ25 splice variant thereof, more preferably, a Cav3.2 isoform protein and/or its δ25 splice variant thereof, in a tissue sample.

In one embodiment, the tissue sample comprises a sample recovered through a biopsy. Furthermore the detection of Cav3 isoform and/or δ25B expression in a sample not only serves as a diagnostic for cancer but it also indicates a potential strategy for treating the cancer. In one embodiment a composition comprising an anti-Cav3 protein and /or an anti-δ25B antibody, preferably, an anti-Cav3.1, anti-Cav3.2 or anti-Cav3.3 isoform protein and/or anti-δ25 splice variant thereof more preferably, an anti-Cav3.2 isoform protein and/or anti-δ25 splice variant thereof, is administered to a patient as a means of treating cancer.

In another embodiment, the cancer is prostate cancer. The anti-Cav3. and/or an anti-δ258 antibody can be directly administered to the patient or the antibodies can be coupled to one or more toxic agents as a way to targeting the toxin to the cancer cells a further enhancing the anti-cancer efficacy of the anti-Cav3 and/or an anti-δ25B antibody composition. The anti-cancer compositions of the present invention can be administered using any of the known routes of administration, including, intravenous, intramuscular, intraparenteral, or subcutaneous injection.

Because the antibodies of the present invention are intended for use in humans as diagnostic and therapeutic agents, one embodiment of the present invention is directed to humanized versions of these antibodies. Humanized versions of the antibodies are needed for therapeutic applications because antibodies from non-human species may be recognized as foreign substances by the human immune system and neutralized such that they are less useful. Humanized antibodies are immunoglobulin molecules comprising a human and non-human portion. More specifically, the antigen combining region (variable region) of a humanized antibody is derived from a non-human source (e.g. murine) and the constant region of the humanized antibody is derived from a human source. The humanized antibody should have the antigen binding specificity of the non-human antibody molecule and the effector function conferred by the human antibody molecule. Typically, creation of a humanized antibody involves the use of recombinant DNA techniques.

Still another embodiment of the present invention is directed to synthetic compounds that can inhibit the Ca2+ entry into non-excitable cells and, consequently, inhibit proliferation of several cancer cell lines.

One embodiment of the present invention is directed to a method of inhibiting cancer cell proliferation, preventing, treating or controlling cancers in a patient by administering a therapeutic effective amount of the synthetic compounds that can inhibit the Ca2+ entry into non-excitable cells, particularly, cancer cells.

An aspect of the present invention is to provide methods for inhibiting calcium entry into electrically non-excitable cells with a T type calcium channel selective inhibitor. According to the present invention, non-excitable cells include, but are not limited to, lymphocytes, epithelial cells, connective tissue cells, secretory cells, Jurkat T-cells, MDA-468 cells and PC-3 cells.

Another aspect of the present invention is to provide methods for preventing proliferation of electrically non-excitable cells, with a T type calcium channel selective inhibitor.

Still another aspect of the present invention is to provide methods of treating autoimmune diseases with a T type calcium channel selective inhibitor.

One aspect of the present invention is directed to a method for preventing graft rejections with a T type calcium channel selective inhibitor.

Another aspect of the present invention is directed to a method for preventing apoptosis with a T type calcium channel selective inhibitor.

The present invention is further illustrated by the following non-limiting examples.

### EXAMPLE 1

Extracellular free calcium must enter cancer cells in order for the cells to transit the cell cycle and divide. Consequently, blockade or inhibition of calcium. entry will stop or slow the rate at which cancer cells divide and increase a hosts cancer burden. As shown in the Figure 1A to 1F, mibefradil, which has formula of (18,28)-(2 {[3-(2-b enzimidazolyl)propyl]methylamino} ethyl)-6-fluoro-1,2,3,4-tetrahydro-1-isopropyl-2-naphthylmethoxyacetate dihydrochloride, inhibited stimulated calcium entry concordantly with inhibition of proliferation.

Mibefradil, which is a member of the chemical family of tetrahydronaphthalene derivatives, was added at varying concentrations as indicated in Figure 1A-1F. Cancer cell proliferation was measured by the MTT assay (Promega) using the manufacturer's directions. The rise in the intracellular concentration of calcium, which is a direct indication of calcium entry (Haverstick, D.M. and Gray, L. S. Mol. Biol. Cell 4:173, 1993), was measured and induced by standard methods as described in, for example, (Haverstick, D.M., et al.J. Immunol. 146:3306 1991). The data shown in Figures 1A-1F indicate that mibefradil blocked the calcium entry from the extracellular medium that is necessary for cancer cell division and proliferation. Consequently, mibefradil and pharmaceutical compositions containing it can be administered to hosts in need for the prevention, control, or treatment of cancer and conditions related to cancer.

The three cancer cell lines shown in Figures 1A-1F were derived from. human prostate cancer specimens.

## Claims

1. An antibody that specifically binds to a Cav3 isoform or its δ25 splicing variants thereof.

2. The antibody of claim 1, wherein the Cav3 isoform is a Cav3.2 isoform.

3. The antibody of claim 1, wherein the antibody is a humanized antibody.

4. A pharmaceutical composition comprising an antibody of claim 1 or claim 3 and a pharmaceutically acceptable carrier.

5. An antibody according to any one of claims 1 to 3 or the pharmaceutical composition of claim 4 for use in the treatment of cancer.
